# EUROPEAN PATENT SPECIFICATION

(11) **EP 2 684 047 B1**
(45) Date of publication and mention of the grant of the patent: **17.01.2018**
(21) Application number: 12754982.2
(22) Date of filing: 07.03.2012
(51) Int. Cl.: G01N 33/92, G01N 33/68

(54) **COMPOSITIONS AND METHODS FOR DETERMINING RISK OF A CARDIOVASCULAR OCCLUSIVE EVENT**
ZUSAMMENSETZUNGEN UND VERFAHREN ZUR BESTIMMUNG DES RISIKOS EINES KARDIOVASKULÄREN OKKLUSIONSEREIGNISSES
COMPOSITIONS ET MÉTHODES DE DÉTERMINATION DE RISQUE D'ACCIDENT CARDIOVASCULAIRE OCCLUSIF

(30) Priority: 10.03.2011 US 201161451487 P
(43) Date of publication of application: 15.01.2014
(73) Proprietor: The Regents of the University of California, Oakland, CA 94607 (US)
(72) Inventor: KANE, John P., Hillsborough, California 94010 (US); ISHIDA, Brian, Walnut Creek, California 94597 (US)
(74) Representative: Brasnett, Adrian Hugh
(86) International application number: PCT/US2012/028023
(87) International publication number: WO 2012/122242

(56) References cited:
- JP-A- 2005 030 824
- US-B2- 7 749 729
- Takashi Miida ET AL: "Pref31-high-density lipoprotein increases in coronary artery disease", ClinicalChemistry, 1 January 1992 (1992-01-01), pages 1992-1995, XP055137021, Retrieved from the Internet: URL:http://www.clinchem.org/content/42/12/ 1992.full.pdf
- T. MIIDA: "Analytical performance of a sandwich enzyme immunoassay for prebeta1-HDL in stabilized plasma", THE JOURNAL OF LIPID RESEARCH, vol. 44, no. 3, 16 December 2002 (2002-12-16), pages 645-650, XP055167319, ISSN: 0022-2275, DOI: 10.1194/jlr.D200025-JLR200
- P.M. O'CONNOR ET AL: "Measurement of Prebeta-1 HDL in Human Plasma by an Ultrafiltration-Isotope Dilution Technique", ANALYTICAL BIOCHEMISTRY, vol. 251, no. 2, 1 September 1997 (1997-09-01), pages 234-240, XP055167179, ISSN: 0003-2697, DOI: 10.1006/abio.1997.2258
- LIN T GUEY ET AL: "Relation of Increased Prebeta-1 High-Density Lipoprotein Levels to Risk of Coronary Heart Disease", AMERICAN JOURNAL OF CARDIOLOGY, CAHNERS PUBLISHING CO., NEWTON, MA, US, vol. 108, no. 3, 18 March 2011 (2011-03-18), pages 360-366, XP028236782, ISSN: 0002-9149, DOI: 10.1016/J.AMJCARD.2011.03.054 [retrieved on 2011-04-27]
- MIIDA ET AL.: 'Pre beta 1-high-density lipoprotein increases in coronary artery disease.' CLIN CHEM. vol. 42, no. 12, December 1996, pages 1992 - 1995, XP055137021
- FIELDING ET AL.: 'Unique epitope of apolipoprotein A-I expressed in pre-beta-1 high-density lipoprotein and its role in the catalyzed efflux of cellular cholesterol.' BIOCHEMISTRY vol. 33, no. 22, 07 June 1994, pages 6981 - 6985, XP002924323

### SEQUENCE LISTING

<110> Kane, John P.
   Ishida, Brian
<120> Compositions and Methods for Determining
   Risk of a Cardiovascular Occlusive Event
<130> UCSF-193WO
<150> 61/451,487
   <151> 2011-03-10
<160> 1
<170> FastSEQ for Windows Version 4.0
<210> 1
   <211> 267
   <212> PRT
   <213> Homo sapiens
<400> 1

## Description

### BACKGROUND

Cardiovascular disease (CVD) is the general term for heart and blood vessel diseases, including atherosclerosis, coronary artery disease, cerebrovascular disease, aorto-iliac disease, and peripheral vascular disease. Individuals with CVD may develop a number of complications, such as myocardial infarction, stroke, angina pectoris, transient ischemic attacks, congestive heart failure, aortic aneurysm, and death.

Levels of the acute phase reactant C-reactive protein (CRP) have been used to determine an individual's risk of developing a cardiovascular disorder. However, CRP may be found in the blood of individuals with inflammation due to causes other than CVD; as such, the value of CRP as a diagnostic or prognostic tool may be limited.

Atherothrombotic disease of the coronary circulation is widely recognized to be multifactorial. The process is initiated by the insudation of atherogenic lipoproteins containing the B apolipoproteins into the subintima, where oxidative modification of the lipoproteins initiates free radical-mediated inflammation, attracting macrophages and other inflammatory cells. High-density lipoproteins (HDL) exert a number of atheroprotective activities, the best understood of which is the promotion of efflux of cholesterol (and certain phospholipid species) from macrophages in the artery wall. This process is mediated by several lipid transporters, among which the ATP cassette transporter, ABCA1, appears to be the most active. This process involves efflux of unesterified cholesterol to a relatively lipid-poor molecular species of HDL, preβ-1 HDL.

There is a need in the art for methods of determining an individual's risk of developing a cardiovascular occlusive event. There is a need in the art for methods of determining an individual's risk of having a myocardial infarction.

### Literature

Asztalos et al. (1993) Biochim. Biophys. Acta 1169:291; Asztalos et al. (2004) Arterioscler. Thromb. Vasc. Biol. 24:2181; Asztalos et al. (2005) Arterioscler. Thromb. Vasc. Biol. 28:575; Asztalos et al. (2008) Metab. Clin. Exp. 57:77; Duong et al. (2008) J. Lipid Res. 49:1006; Giunta et al. (1992) Ann. NY Acad. Sci. 673:342; Hattori et al. (2004) Clin. Chem. 50:589; Khera et al. (2011) New Engl. J. Med. 364:127; Kunitake et al. (1985) J. Lipid Res. 26:549; Miida et al. (1996) Clin. Chem. 42:1992; Okazaki et al. (2006) Clin. Chem. 52:2049; Otvos et al. (2006) Circulation 113:1556; Semprini et al. (1992) Arch. Gerontol. Geriatri. 15 Suppl.1:325; Sethi et al. (2010) Clin. Chem. 56:1128; Tashiro et al. (2009) Atheroscler. 204:595.

JP 2005 030824 relates to the inspection method of acute crown syndrome and its symptoms. Miida et al, Clinical Chemistry, 1 January 1992, pages 1992-1995 reports finding that preβ1 high density lipoprotein concentrations increase in coronary artery disease.

### SUMMARY

The present invention provides a method of identifying an individual at risk of having a myocardial infarction, the method comprising: determining the level of pre-beta-1 high density lipoprotein (preβ-1 HDL) in a biological sample from the individual, wherein a level of preβ-1 HDL that is higher than a normal control level indicates that the individual is at increased risk of having a myocardial infarction. The present disclosure also sets out ("provides") a number of other methods and products, e.g., kits, as discussed herein below which may facilitate understanding of the present invention, which is defined by the claims.

The present disclosure provides methods for assessing risk of a cardiovascular occlusive event in an individual. The methods find use in diagnostic and prognostic applications. The present disclosure provides a method for measuring the level of preβ-1 high density lipoprotein in a biological sample; and also provides kits for use in carrying out the method.

### BRIEF DESCRIPTION OF THE DRAWINGS

Figure 1 provides a schematic representation of the preβ-1 HDL cycle.
Figure 2 provides an amino acid sequence of apoA-I (SEQ ID NO:1).

### DEFINITIONS

The terms "subject," "individual," and "patient" are used interchangeably herein to refer to a human.

A "biological sample" encompasses a variety of sample types obtained from an individual and can be used in a diagnostic or monitoring assay. The definition encompasses blood, blood products (e.g., serum; plasma), and other liquid samples of biological origin, solid tissue samples such as a biopsy specimen or tissue cultures or cells derived therefrom and the progeny thereof. The definition also includes samples that have been manipulated in any way after their procurement, such as by treatment with reagents, solubilization, or enrichment for certain components, such as lipoproteins (e.g., preβ-1 HDL). A biological sample can be fresh, or can be frozen or otherwise treated. In some embodiments, a biological sample may include compounds which are not naturally intermixed with the sample in nature such as preservatives, anticoagulants, buffers, fixatives, nutrients, antibiotics, and the like. In some embodiments, a biological sample may include compounds which are not naturally intermixed with the sample in nature such as preservatives, anticoagulants, buffers, fixatives, nutrients, antibiotics, or the like The term "biological sample" encompasses a clinical sample, and also includes cells in culture, cell supernatants, cell lysates, urine, saliva, serum, plasma, biological fluid, and tissue samples.

"Isolated" refers to an entity of interest that is in an environment different from that in which the compound may naturally occur. "Isolated" is meant to include compounds that are within samples that are substantially enriched for the compound of interest and/or in which the compound of interest is partially or substantially purified.

By "purified" is meant a compound of interest (e.g., preβ-1 HDL) has been separated from components that accompany it in nature. "Purified" can also be used to refer to a compound of interest separated from components that can accompany it during manufacture or preparation. In some embodiments, a compound is substantially pure when it is at least 50% to 60%, by weight, free from organic molecules with which it is naturally associated or with which it is associated during purification or synthesis. In some embodiments, the preparation is at least 85%, at least 90%, at least 95%, or at least 99%, by weight, of the compound of interest. Purity can be measured by any appropriate method, e.g., chromatography, mass spectroscopy, high performance liquid chromatography analysis, gel electrophoresis, an immunological method, fast protein liquid chromatography, etc.

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described.

It must be noted that as used herein and in the appended claims, the singular forms "a," "an," and "the" include plural referents unless the context clearly dictates otherwise. Thus, for example, reference to "a biological sample" includes a plurality of such biological samples and reference to "the pre-beta-1 high density lipoprotein" includes reference to one or more pre-beta-1 high density lipoproteins and equivalents thereof known to those skilled in the art, and so forth. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all sub-combinations of the various embodiments and elements thereof are also specifically embraced by the present invention and are disclosed herein just as if each and every such sub-combination was individually and explicitly disclosed herein.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### DETAILED DESCRIPTION

The present disclosure provides methods for assessing risk of a cardiovascular occlusive event in an individual. The methods find use in diagnostic and prognostic applications. The present disclosure provides a method for measuring the level of preβ-1 high density lipoprotein in a biological sample; and also provides kits for use in carrying out the method.

The particle known as preβ-1 HDL has a molecular weight of about 60 kDa, and includes apolipoprotein A-I (apoA-I) and no other apolipoproteins. Preβ-1 HDL particles were initially identified electrophoretically; the "preβ-1" designation reflects the particle's electrophoretic mobility. Kunitake et al. (1985) J. Lipid Res. 26:549. Thus, preβ-1 HDL has a well-established characteristic electrophoretic mobility.

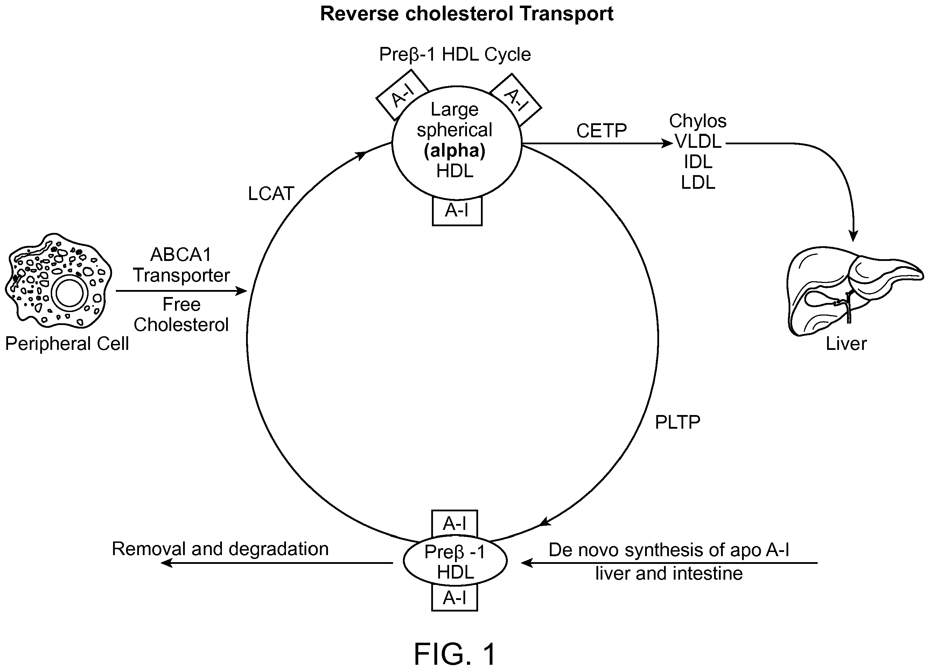

As shown schematically in Figure 1, preβ-1 HDL is formed as newly synthesized apoA-I enters the plasma compartment, and as a substrate or byproduct in the interconversion of several HDL species. After the acquisition of unesterified cholesterol, it becomes the substrate for lecithin-cholesterol acyl transferase (LCAT), resulting in esterification of free cholesterol with a fatty acyl residue derived from lecithin. As the relatively hydrophobic cholesteryl esters accumulate, they form a central core in a pseudomicellar organization, accreting apolipoproteins on the surface to form larger spherical HDL particles. The preβ-1 HDL particles are subsumed into the spherical species as they organize. Thus, the steady state level of preβ-1 HDL in plasma reflects (1) rates of generation via de novo synthesis and that resulting from transfer of cholesteryl esters from HDL to acceptor lipoproteins, and (2) removal by catabolism and conversion to larger HDL species by the action of LCAT.

Data presented in the Examples, below, show that, in a study involving a large, well-characterized clinical cohort, preβ-1 HDL is an independent predictor of myocardial infarction (MI). In some earlier studies, preβ-1 HDL was detected using 2-dimensional gel electrophoresis; however, because a common sample of purified preβ-1 HDL was not used as a standard, absolute quantification was not possible. The study described in the Examples was based on quantitative analysis of preβ-1 HDL, using purified preβ-1 HDL as a standard. Accordingly, the present disclosure provides a method of determining the risk that an individual will have a cardiovascular occlusive event, such as an MI.

### METHODS OF DETERMINING CARDIOVASCULAR DISEASE RISK

The present disclosure provides methods of identifying individuals who are at risk of having a cardiovascular occlusive event (e.g., an MI). The methods generally involve determining the level of pre-beta-1 high density lipoprotein (preβ-1 HDL) in a biological sample obtained from an individual being tested. A level of preβ-1 HDL that is higher than a normal control level indicates that the individual is at increased risk of having a cardiovascular occlusive event. The present disclosure provides methods for determining cardiovascular occlusive event risk in an individual, where a measured level of preβ-1 HDL in a biological sample obtained from the individual provides an indication of the risk that the individual has or will develop a cardiovascular occlusive event.

As an example, where the level of preβ-1 HDL in a biological sample obtained from an individual is from about 15% to about 25% higher, from about 25% to about 50% higher, from about 50% to about 2-fold higher, from about 2-fold to about 5-fold higher, or more than 5-fold higher, than a normal control value, the individual is considered at risk of developing cardiovascular occlusive event, e.g., the individual is considered at risk of having a myocardial infarction.

In some cases, a biological sample obtained from an individual for testing according to a method of the present disclosure is stored below 12°C immediately (e.g., within 1 minute to about 15 minutes) after collection from the individual. For example, the biological sample is stored below 12°C, below 10°C, below 8°C, below 6°C, or at or below 4°C. The biological sample can be stored frozen until tested according to a method of the present disclosure.

### Pre-beta-1 high density lipoprotein

Pre-beta-1 high density lipoprotein (preβ-1 HDL) is an approximately 60 kDa to 70 kDa particle that is lipid poor, and includes one or two apolipoprotein A-I (apoA-I) molecules, but no other lipoproteins or apolipoproteins. Preβ-1 HDL has a characteristic electrophoretic mobility on agarose and/or denaturing or non-denaturing polyacrylamide gels. A preβ-1 HDL particle can include three to five phospholipid molecules and one or zero to three cholesterol molecules. A preβ-1 HDL particle can have an effective hydrodynamic diameter of 7.5 ± 0.4 nm. Duong et al. (2008) J. Lipid Res. 49:1006.

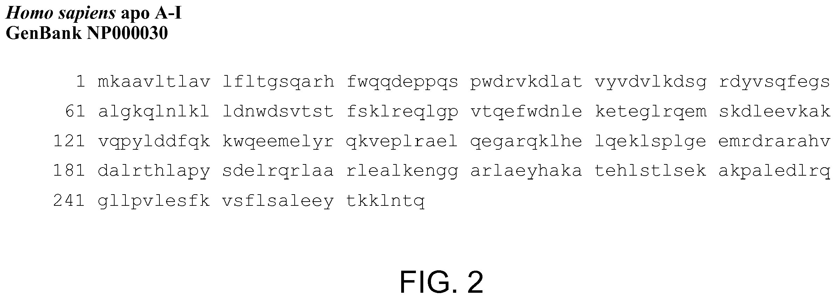

ApoA-I is a polypeptide comprising an amino acid sequence having at least about 95%, at least about 98%, at least about 99%, or 100%, amino acid sequence identity with from about 150 to about 200, from about 200 to about 250, or from about 250 to 267 contiguous amino acids of the amino acid sequence depicted in Figure 2. ApoA-I can comprise an amino acid sequence as depicted in Figure 2, or naturally-occurring variants (polymorphisms) of the amino acid sequence depicted in Figure 2.

### Control values

Levels of the preβ-1 HDL in a biological sample obtained from a test subject are compared to a normal control value(s) or range of normal control values. The control value can be based on levels of preβ-1 HDL in comparable samples (e.g., blood, plasma, or serum sample, or other biological sample) obtained from a control population, e.g., the general population or a select population of human subjects. For example, the select population may be comprised of apparently healthy subjects, e.g., individuals who have not previously had any signs or symptoms of cardiovascular disease, including a cardiovascular occlusive event. Apparently healthy individuals also generally do not otherwise exhibit symptoms of disease. In other words, such individuals, if examined by a medical professional, would be characterized as healthy and free of symptoms of disease.

The control value can take a variety of forms. The control value can be a single cut-off value, such as a median or mean. A normal control value can be a normal control range. The control value can be established based on comparative groups such as where the risk in one defined group is double the risk in another defined group. The control values can be divided equally (or unequally) into groups, such as a low risk group, a medium risk group and a high-risk group, or into quadrants, the lowest quadrant being individuals with the lowest risk the highest quadrant being individuals with the highest risk, and the test subject's risk of having a cardiovascular occlusive event (e.g., a test subject's risk of MI) can be based upon which group his or her test value falls.

### Biological Samples

Suitable biological samples useful for predicting or monitoring cardiovascular disease in a subject or for assessing the effect of therapeutic agents on subjects with cardiovascular disease include, but are not limited to, whole blood samples, and blood fractions (also referred to as "blood products"), where suitable blood fractions include, but are not limited to, serum and plasma. The biological sample can be fresh blood or stored blood (e.g. in a blood bank) or blood fractions. The biological sample can be a blood sample expressly obtained for an assay of the present disclosure or a blood sample obtained for another purpose which can be subsampled for an assay of the present disclosure.

In one embodiment, the biological sample is whole blood. Whole blood can be obtained from the subject using standard clinical procedures. In another embodiment, the biological sample is plasma. Plasma can be obtained from whole blood samples by centrifugation of anticoagulated blood. Such process provides a buffy coat of white cell components and a supernatant of the plasma. In another embodiment, the biological sample is serum.

The sample can be pretreated as necessary by dilution in an appropriate buffer solution, heparinized, concentrated if desired, or fractionated by any number of methods including but not limited to ultracentrifugation, fractionation by fast protein liquid chromatography (FPLC), or precipitation. Any of a number of standard aqueous buffer solutions, employing one of a variety of buffers, such as phosphate, Tris, or the like, at physiological pH can be used.

### Subjects

Individuals who are to be tested using a method of the present disclosure include individuals who have never experienced an MI; individuals who have had at least one MI; individuals who have experienced one or more typical symptoms of cardiovascular disease; individuals who have experienced an atypical symptom of cardiovascular disease; smokers; non-smokers; individuals aged 50 years or older; individuals who have a body mass index greater than about 25 kg/m², or greater than about 30 kg/m²; individuals with a dyslipidemia; individuals who have a risk factor for coronary artery disease (CAD), where the risk factor is, e.g., higher than normal range of low density lipoprotein (LDL), and the like; individuals with hypertension; individuals with type 2 diabetes; and apparently healthy individuals. An individual can be male or female. In some instances, the individual is a female who has experienced an atypical symptom of cardiovascular disease, e.g., a symptom not normally associated with cardiovascular disease. Individuals who are to be tested using a method of the present disclosure also include the general population.

### Determining a level of preβ-1 HDL

A method of determining risk of a cardiovascular occlusive event (e.g., MI risk), as described herein, involves quantitatively determining the level of preβ-1 HDL in a biological sample. Methods of determining the level of preβ-1 HDL that are suitable for use in a subject method include quantitative methods. For example, Western blotting of a 2-dimensional electrophoretogram, e.g., in the absence of internal pre-β1 HDL standards, may not be suitable, as such a method is not quantitative. Quantitative methods can include an internal standard.

### Ultrafiltration-isotope dilution method

One non-limiting example of a suitable method is an ultrafiltration-isotope dilution method. See, e.g., O'Connor et al. (1997) Anal. Biochem. 251:234. For example, preβ-1 HDL is separated from larger diameter HDL particles by low velocity centrifugation in ultrafilters, then measured by application of the isotope dilution principle. As an example, standard prebeta-1 HDL of greater than 99% purity, prepared from fresh normal human plasma by selected affinity immunosorption and preparative electrophoresis is labeled with a tritium adduct. A precisely measured amount is added to the patient's plasma sample. The content of apoA-I in the ultrafiltrate is measured by enzyme-linked immunosorbent assay (ELISA) for calculation of its specific activity.

As one non-limiting example, an ultrafiltration-isotope dilution method can be carried out as follows. Tritium-labeled prebeta-1 HDL tracer is added to a plasma sample (e.g., 50 µl of specific activity 8-9 x 10⁻³ cpm/µg apoAI protein is added to a 1.0-ml sample of fresh or thawed plasma), to generate a labeled plasma sample. A 400-µL aliquot of the labeled plasma sample is transferred to each of two centrifugal ultrafilters (e.g., Microcon 100, Amicon Corp., Cambridge, MA), and centrifuged at 1°C-4°C at 300 x g in a refrigerated microcentrifuge rotor in which 18 tubes rest at a 30° angle (e.g., Eppendorf Model 5402). After 45 minutes, no more than 50 µl will have been filtered. Adjustment of centrifugation time can be made such that no more than 50µl is filtered, so as to provide for uniform ultrafiltering of all samples. The specific activity of the apoA-I in the ultrafiltrate is determined by scintillation counting, and by enzyme-linked immunosorbent assay.

Purification of preβ-1 HDL for use as a tracer can be carried out as follows. ApoA-I proteins are isolated from plasma by selected affinity immunosorption. McVicar et al. (1984) Proc. Natl. Acad. Sci. USA 81:1356. Essentially, a plasma sample is applied to an anti-apo A-I column, operated under a nitrogen atmosphere. The column comprises apoA-I-specific antibody immobilized on an insoluble support, such as agarose beads or other suitable insoluble support, such that apoA-I-containing lipoproteins present in the applied plasma sample are bound to the immobilized anti-apoA-I antibody. The bound apo A-I-containing lipoproteins are eluted with 3 M acetic acid, generating a column eluate; and the eluate is neutralized to pH 7.0 with Trisma base (3 M). Residual trace albumin and immunoglobulins are removed from the column eluate by passage over anti-albumin and anti-IgG immunosorption columns, generating a subtracted column eluate. The prebeta lipoproteins present in the subtracted column eluate are then separated from other HDL by electrophoresis in 1 cm x 40 cm x 10 cm starch blocks in 0.05 M sodium barbital buffer, pH 8.4. Kunitake et al. (1985) J. Lipid Res. 26:549. Following electrophoresis, beginning at the origin (sample application end) of the gel, 1-cm slices of starch are extracted in 0.15 M NaCl with 0.05 M phosphate containing 0.08% EDTA at pH 7.4 and the apoA-I content of each fraction is detected by rate immunonephelometry on a Beckman ICS Analyzer II immunochemistry system. The high-molecular-weight prebeta-2 and prebeta-3 HDL are excluded by pooling only the gel eluates (gel fractions) from the cathodic upswing of the prebeta HDL peak to its apex. The pooled gel eluate is concentrated to between 0.2 and 0.5 mg/ml by diafiltration in a Micro-ProDiCon vacuum dialysis concentrator, generating a concentrated eluate product. Purity of the product is assessed by electrophoresis of a sample of the concentrated eluate (where the sample contains 10-20 µg protein) in a nondenaturing 3-34% polyacrylamide gradient gel to determine that the apparent molecular weight is about 67 kDa. The purity of the product is also assessed by electrophoresis in a 5-25% sodium dodecyl sulfate (SDS) polyacrylamide gradient gel immunoblotted with anti-apoA-I antibodies and stained with Coomassie blue. The concentrated eluate product is also examined by fast protein liquid chromatography (FPLC). Prebeta mobility is verified by vertical agarose gel electrophoresis. Asztalos et al. (1993) Biochim. Biophys. Acta 1169:301. This purified preβ-1 HDL can be used as a tracer in the above-described ultrafiltration-isotope dilution method.

Labeling of the tracer preβ-1 HDL with tritium can be carried out as described, or using any conventional method. See, e.g., O'Connor et al. (1997) *supra.*

Immunoassay of apoA-I in plasma and in the ultrafiltrate can be carried out using an ELISA assay as described in O'Connor et al. (1997) *supra,* or using any other known method. To calculate the total mass of preβ-1 HDL per plasma sample, the following equation can be used:

(counts applied to ultrafilter) ÷ (specific activity of the ultrafiltrate) = mass of prebeta-1 HDL in sample.

### Immunofixation method

Another example of a suitable method is an immunofixation method, as described in detail below.

### Sandwich enzyme immunoassay

Also suitable for use is a sandwich enzyme immunoassay, such as an assay described in Miyazaki et al. ((2000) J. Lipid Res. 41:2083), or Miida eta 1. ((2003) J. Lipid Res. 44:645).

### Generating a report

A subject method can include generating a report that provides an indication of an individual's assessed risk of a cardiovascular occlusive event (e.g., risk of MI).

In some embodiments, a subject method of determining an individual's risk of a cardiovascular occlusive event (e.g., risk of MI) involves generating a report. Such a report can include information such as a predicted risk that the patient will have a cardiovascular occlusive event, e.g., an MI; a recommendation regarding further evaluation; a recommendation regarding therapeutic drug and/or device intervention; and the like.

For example, the methods disclosed herein can further include a step of generating or outputting a report providing the results of a subject risk assessment, which report can be provided in the form of an electronic medium (e.g., an electronic display on a computer monitor), or in the form of a tangible medium (e.g., a report printed on paper or other tangible medium). An assessment as to the likelihood can be referred to as a "risk report" or, simply, "risk score." A person or entity that prepares a report ("report generator") may also perform steps such as sample gathering, sample processing, and the like. Alternatively, an entity other than the report generator can perform steps such as sample gathering, sample processing, and the like. A risk assessment report can be provided to a user. A "user" can be a health professional (e.g., a clinician, a laboratory technician, a physician (e.g., a cardiologist), etc.).

A subject report can further include one or more of: 1) service provider information; 2) patient data; 3) data regarding the level of preβ-1 HDL; 4) follow-up evaluation recommendations; 5) therapeutic intervention recommendations; and 6) other features.

### Further evaluation

Based on detection of a level of preβ-1 HDL, and/or based on a report (as described above), a physician or other qualified medical personnel can determine whether further evaluation of the test subject (the patient) is require. Further evaluation can include, e.g., angiogram; echocardiogram; electrocardiogram; a test for blood (or blood product, such as plasma or serum) triglyceride levels; a test for blood (or blood product, such as plasma or serum) low density lipoprotein levels; a test for cardiac protein markers (e.g., cardiac troponin, etc.) present in blood or a blood fraction such as plasma or serum; and the like.

### Therapeutic intervention

Based on detection of a level of preβ-1 HDL, and/or based on a report (as described above), a physician or other qualified medical personnel can determine whether appropriate therapeutic intervention is advised, e.g., in order to reduce the risk that the individual will experience a cardiovascular occlusive event, such as an MI.

Therapeutic intervention includes drug-based therapeutic intervention, device-based therapeutic intervention, and surgical intervention. Drug-based therapeutic intervention includes, an anti-inflammatory agent, an antithrombotic agent, an anti-platelet agent, a fibrinolytic agent, a lipid reducing agent, a direct thrombin inhibitor, a glycoprotein IIb/IIIa receptor inhibitor, an agent that binds to cellular adhesion molecules and inhibits the ability of white blood cells to attach to such molecules, a calcium channel blocker, a beta-adrenergic receptor blocker, a cyclooxygenase-2 inhibitor, and an angiotensin system inhibitor.

Device-based therapeutic intervention includes, e.g., installation of an intravascular stent; balloon angioplasty; and the like. Surgical intervention includes, e.g., arterial bypass surgery.

### METHOD OF MEASURING A LEVEL OF PRE-BETA-1 HDL IN A BIOLOGICAL SAMPLE

The present disclosure provides an immunofixation method of measuring the level of preβ-1 HDL in a biological sample. The method generally involves electrophoretically separating components of the biological sample in a gel; and contacting the gel with an antibody specific for apoA-I, e.g., antibody that specifically recognizes apoA-I present in pre1-HDL. The antibody specific for apoA-I enters the gel, and forms complexes with apoA-I-containing molecules in the gel. Anti-apoA-I/apoA-I-containing molecule complexes (for simplicity, referred to as "apoA-I-antibody complexes") become trapped within the gel matrix, and are then detected. The level of detected apoA-I-antibody complexes are compared with the level of purified preβ-1 HDL (or preβ-1 HDL surrogate) standards present in the gel, thereby providing the level of preβ-1 HDL present in the biological sample.

A biological sample is applied to a gel, such as a polyacrylamide gel or an agarose gel, and can be denaturing or non-denaturing. Denaturing gels can include, e.g., sodium dodecyl sulfate. The gel can be a gradient gel, where the gel has a lower concentration of the gel matrix (polyacrylamide; agarose; etc.) at the sample application end of the gel and a higher concentration of gel matrix at the opposite end of the gel. The concentration of gel matrix (polyacrylamide; agarose; etc.) can be uniform (e.g., 5%, 7%, 10%, or other concentration of gel matrix). For example, a polyacrylamide gel can be a 5-25% gradient gel, or other suitable concentration gradient of polyacrylamide.

A biological sample(s) is applied to a sample application end of the gel. In addition, in separate locations from the biological sample, known amounts of purified preβ-1 HDL are added to the gel to provide a standard curve, which allows for quantitation of the preβ-1 HDL in the biological sample. The biological sample(s) and the preβ-1 HDL standards are electrophoresed, i.e., an electric field is applied to the gel. During electrophoresis, components present in the biological sample are separated from one another. Electrophoresis is carried out, as is standard in the art, with the gel in a suitable buffer such as a Tris-based buffer, a phosphate buffer, etc. Electrophoresis is carried out for from about 30 minutes to about 4 hours or more, e.g., from about 30 minutes to about 1 hour, from about 1 hour to about 2 hours, from about 2 hours to about 3 hours, or from about 3 hours to about 4 hours, or more than 4 hours. The current applied can be from 50 volts to 300 volts, or any other suitable current. In some embodiments, one-dimensional gel electrophoresis is carried out. In other embodiments, two-dimensional gel electrophoresis is carried out.

After electrophoresis, the gel is contacted with an antibody that specifically binds apoA-I. The antibody enters the gel and forms complexes with apoA-I-containing molecules present in the biological sample, thereby generating apoA-I-antibody complexes. The apoA-I-antibody complexes are large and are therefore "fixed" within the gel, e.g., the apoA-I-antibody complexes are not readily removed from the gel matrix. The fixation process can occur when antibody comes in contact with apoA-I-containing molecules at temperatures ranging from 3°C to 37° C over a period of time ranging from several minutes (e.g., from about 5 minutes to about 120 minutes) to hours (e.g., from about 2 hours to about 4 hours, from about 4 hours to about 6 hours, from about 6 hours to about 8 hours), or longer than 8 hours. The antibody can be applied in a liquid medium in the form of purified antibody suspended in an appropriate buffer solution or as antisera (e.g. monospecific goat antiserum) at neat or diluted in an appropriate buffer solution. The antibody-containing medium may be used as such or used with additives, including but not limited to, a non-ionic detergent (e.g., Triton X-100, Tween-20, and NP-40), a protease inhibitor, and the like.

A suitable anti-apoA-I antibody includes a polyclonal antibody, a monoclonal antibody, and an antigen-binding fragment (e.g., a Fv, scFv, Fab, F(ab')2, or Fab' fragment). Polyclonal antibody can include a population of antibodies, where the population comprises antibody specific for apoA-I, and can also include antibodies of other specificities. For example, a polyclonal antibody can include a population of antibodies, where the proportion of the population that are specific for apoA-I is from about 50% to about 75%, from about 75% to about 80%, from about 80% to about 90%, or greater than 90% (e.g., from 90% to 95%, from 95% to 98%, or greater than 98%). A suitable anti-apoA-I antibody is immunospecific, e.g., does not substantially bind to an apolipoprotein other than apoA-I, e.g., binds to an apolipoprotein other than apoA-I, if at all, with an affinity of less than 10⁻⁷ M, e.g., binds to an apolipoprotein other than apoA-I, if at all, with an affinity of 10⁻⁶ M, 10⁻⁵ M, or less than 10⁻⁵ M. A suitable anti-apoA-I antibody binds apoA-I with an affinity of at least about 10⁻⁷ M, e.g., from about 10⁻⁷ M to about 5 x 10⁻⁸ M, from about 5 x 10⁻⁸ M to about 10⁻⁸ M, or from about 5 x 10⁻⁸ M to about 10⁻⁹ M. In some cases, the antibody is specific for apoA-I present in preβ-1 HDL, e.g., the antibody binds to apoA-I present in preβ-1 HDL and not to apoA-I when present in other (non-preβ-1) HDL particles.

Suitable antibodies include an anti-apoA-I antibody that comprises a detectable label. Suitable detectable labels include any composition detectable by spectroscopic, photochemical, biochemical, immunochemical, electrical, optical or chemical means. Suitable include, but are not limited to, fluorescent labels (e.g., fluorescein isothiocyanate, texas red, rhodamine, a green fluorescent protein, a red fluorescent protein, a yellow fluorescent protein, and the like), radiolabels (e.g., ³H, ¹²⁵I, ³⁵S, ¹⁴C, or ³²P), and enzymes (e.g., horse radish peroxidase, alkaline phosphatase, luciferase, and other enzymes that act on a substrate to produce a product that can be detected by fluorometric, colorimetric, or spectrophotometric means).

Preβ-1 HDL standards, or a surrogate, are added to the gel, and are applied at sample application sites separate from the biological sample application site. Purified preβ-1 HDL standards can include 3, 4, 5, 6, or more than 6 known quantities of preβ-1 HDL. As one non-limiting example, a set of preβ-1 HDL standards could include 0.1 µg, 0.5 µg, 1.0 µg, 1.5 µg, 2.0 µg, and 5.0 µg purified preβ-1 HDL. As another non-limiting example, a set of preβ-1 HDL standards could include 0.08 µg, 0.16 µg, 0.33 µg, 0.67 µg, and 1.33 µg purified preβ-1 HDL. A set of preβ-1 HDL standards can be in solution, in concentrations of, e.g., 5 mg/dL, 10 mg/dL, 25 mg/dL, 50 mg/dL, 75 mg/dL, and 100 mg/dL. As another example, a set of preβ-1 HDL standards can be in solution, in concentrations of, e.g., 8 mg/dL, 17 mg/dL, 33 mg/dL, 67 mg/dL and 133 mg/dL.

Preβ-1 HDL can be purified using any of a variety of methods. For example, preβ-1 HDL can be purified using selected immunosorption, e.g., as described in McVicar et al. ((1984) Proc. Natl. Acad. Sci. USA 81:1356), in which apoA-I-containing lipoproteins present in the applied plasma sample are bound to immobilized anti-apoA-I antibody, then eluted. Preβ-1 HDL can also be purified by a fast protein liquid chromatography method, or by a block electrophoresis method. Preβ-1 HDL can be purified under non-denaturing conditions.

As an alternative to purified preβ-1 HDL, urea-disrupted HDL can be used as a surrogate electrophoretic marker and apoAI mass calibrator for preβ-1 HDL. As an example, urea-disrupted HDL is prepared from HDL prepared by sequential potassium bromide density gradient ultracentrifugation (1.063 < d < 1.21 g/ml) of human plasma obtained from healthy subjects. The isolated HDL is extensively dialyzed against buffer (0.15 M NaCl, 0.001 M Na-EDTA, 0.02% sodium azide). HDL is characterized for total protein content (BioRad DC Kit, bovine serum albumin standard) and its fractional apoAI content determined as the percent area of peaks scanned and integrated (BioRad Chemdoc, Image Lab software) corresponding to the Coomassie blue R-250 stained apoAI band (Mᵣ 25 kDa) on sodium docecyl sulfate-polyacrylamide gel electrophoresis (SDS-PAGE). Stock HDL (appr. 5 mg/ml) solutions are disrupted by the addition of urea buffer (6M urea, 0.02 M Tris, 0.001 M EDTA, 0.02% sodium azide), at temperatures ranging from 15° to 25° C, to effect a final apoAI concentration of 0.133 micrograms/ml. The disrupted HDL solution is serial two-fold diluted in urea buffer to effect solutions of urea-disrupted HDL (apoHDL) containing the apoAI equivalents of 133 mg/dL, 67 mg/dL, 33 mg/dL, 17 mg/dL, and 8 mg/dL.

After electrophoresis, apoA-I-antibody complexes are detected. ApoA-I-antibody complexes can be detected using any convenient method. For example, apoA-I-antibody complexes can be detected by staining the complexes with a conventional stain such as Coomassie blue or silver stain. Alternatively, e.g., where the anti-apoA-I antibody comprises a detectable label, the detectable label can be detected.

In some cases, a step of removing uncomplexed components from the gel is carried out after electrophoresis and before detection of apoA-I-antibody complexes. Removal of uncomplexed molecules can be achieved by, e.g., soaking the gel for from 30 minutes to 3 hours, or longer than 3 hours, in a suitable buffer at temperatures ranging from 3° to 37° C.

Any of a variety of biological samples can be applied to the gel. Generally, the biological sample is a liquid sample. Non-limiting examples of suitable biological samples include blood, a blood fraction such as plasma or serum, and the like.

Once the apoA-I-antibody complexes are detected, apoA-I-antibody complexes formed by preβ-1 HDL present in a biological sample are compared with the level of purified preβ-1 HDL standards (or urea-disrupted HDL surrogate) present in the gel. The comparison allows quantification of the level of preβ-1 HDL present in the biological sample(s). The comparison can be carried out by an individual by visual inspection. Alternatively, the comparison can be carried out by automated means, e.g., using instrumentation that generates a standard curve based on signal provided by the purified preβ-1 HDL standards (or urea-disrupted HDL surrogate) present in the gel, and that compares signal generated by preβ-1 HDL detected in a biological sample with the standard curve, to provide quantification of the preβ-1 HDL present in the biological sample. The "signal" generated by the purified preβ-1 HDL standards (or urea-disrupted HDL surrogate) and by the detected preβ-1 HDL present in a biological sample can be the amount of dye detected (e.g., where apoA-I-antibody complexes are stained with a dye), or the amount of detectable label detected (e.g., where the antibody in the apoA-I-antibody complexes comprises a detectable label).

A non-limiting example of a suitable immunofixation assay is as follows. For example, one-dimensional agarose gel electrophoresis is carried out. Plasma samples and purified preβ-1 HDL standards (known amounts of purified preβ-1 HDL; e.g., 0.1 µg, 0.25 µg, 0.5 µg, 0.75 µg, 1 µg, and 2 µg; or 0.08 µg, 0.17 µg, 0.33 µg, 0.67 µg, and 1.33 µg)) are applied to an agarose gel, and electrophoresis is carried out to separate the components of the applied plasma sample. After electrophoresis, a solution containing anti-apoA-I antibody is spread over the surface of the gel, and allowed to soak into the gel for 4 hours at room temperature, generating an immunofixed gel. The anti-apoA-I antibody forms a complex with apoA-I-containing lipoproteins in the gel. The antibody-apoA-I lipoprotein complexes are large complexes that do not readily elute from the gel, and are therefore fixed within the gel. Uncomplexed material is washed out of the immunofixed gel by soaking the immunofixed gel in buffer overnight. The washed, immunofixed gel is then dried, then stained with an appropriate staining agent, such as Coomassie blue, to allow visualization of the complexes. Preβ-1 HDL is identified as an apoA-I-containing lipoprotein that has the same electrophoretic mobility as the purified preβ-1 HDL standard applied to the gel. Quantitation of preβ-1 HDL in a plasma sample is achieved by comparison with the purified standard preβ-1 HDL applied to the gel. This method can also be carried out in two-dimensional gel electrophoresis.

### METHODS OF ASSESSING IMPACT OF THERAPY ON PRE-BETA-1 HDL METABOLISM

The present disclosure provides methods of determining the impact of a therapeutic agent or a therapeutic regimen for treating a CVD or other disorder on pre-β1 HDL metabolism. The methods generally involve determining the level of preβ-1 HDL in a biological sample from an individual undergoing treatment for a CVD or other disorder.

The methods are useful for, e.g., assessing the effect of an experimental agent or therapeutic regimen on preβ-1 HDL metabolism, e.g., in the course of a clinical trial. If the level of preβ-1 HDL increases following administration of the therapeutic agent or therapeutic regimen, the therapeutic agent or therapeutic regimen is considered to elevate risk of a cardiovascular occlusive event in the individual.

In some embodiments, a subject method involves determining the level of preβ-1 HDL in a biological sample obtained from an individual at a first time point; and determining the level of preβ-1 HDL in a biological sample obtained from the individual at at least a second time point, where the second time point is later than the first time point. In some cases, the first time point is before the start of a therapeutic regimen for treating a CVD; and the second time point is after the start of the therapeutic regimen. In other cases, the first time point is a first time point after the start of a therapeutic regimen for treating a CVD; and the second time point is a second time point after the start of the therapeutic regimen. The interval between any two time points (e.g" between a first time point and a second time point; between a second time point and a third time point; etc.) is from about 24 hours to about 2 days, from about 2 days to about 7 days, from about 1 week to about 2 weeks, from about 2 weeks to about 4 weeks; from about 1 month to about 3 months; or from about 3 months to about 6 months; or longer than 6 months.

Where the level of preβ-1 HDL increases after treatment with a therapeutic regimen, the therapeutic regimen may be re-evaluated.

### KITS

The present disclosure provides kits and systems for carrying out an immunofixation method, as described above, for determining a level of preβ-1 HDL in a biological sample obtained from an individual.

In some embodiments, a subject kit includes: a) an antibody reagent that specifically binds apoA-I (e.g., apoA-I present in preβ-1 HDL); b) a purified preβ-1 high density lipoprotein (preβ-1 HDL) control; and c) components for carrying out gel electrophoresis of a biological sample.

In some embodiments, a subject kit includes: a) an antibody reagent that specifically binds apoA-I (e.g., apoA-I present in preβ-1 HDL, and not to apoA-I present in other (non-preβ-1) HDL particles); and a serum or plasma standard for preβ-1 HDL. A serum or plasma standard for preβ-1 HDL can include pooled sera from human subjects, where the pool is a "high preβ-1 HDL pool." A "high preβ-1 HDL pool" comprises pooled serum from human subjects that is determined to have a high level of preβ-1 HDL using purified preβ-1 HDL as a standard, and compared with a "low preβ-1 HDL pool" comprising pooled serum from human subjects determined to have a low level of preβ-1 HDL using purified preβ-1 HDL as a standard.

A suitable antibody reagent that specifically binds apoA-I (an anti-apoA-I antibody) includes a polyclonal antibody, a monoclonal antibody, and an antigen-binding fragment (e.g., a Fv, scFv, Fab, F(ab')2, or Fab' fragment). A suitable anti-apoA-I antibody is immunospecific, e.g., does not substantially bind to an apolipoprotein other than apoA-I, e.g., binds to an apolipoprotein other than apoA-I, if at all, with an affinity of less than 10⁻⁷ M, e.g., binds to an apolipoprotein other than apoA-I, if at all, with an affinity of 10⁻⁶ M, 10⁻⁵ M, or less than 10⁻⁵ M. A suitable anti-apoA-I antibody binds apoA-I with an affinity of at least about 10⁻⁷ M, e.g., from about 10-⁷M to about 5 x 10⁻⁸ M, from about 5 x 10⁻⁸ M to about 10⁻⁸ M, or from about 5 x 10⁻⁸ M to about 10⁻⁹ M. In some cases, the antibody is specific for apoA-I present in preβ-1 HDL, e.g., the antibody binds to apoA-I present in preβ-1 HDL and not to apoA-I present in any other HDL electrophoretic fraction.

Suitable antibodies include an anti-apoA-I antibody that comprises a detectable label. Suitable detectable labels include any composition detectable by spectroscopic, photochemical, biochemical, immunochemical, electrical, optical or chemical means. Suitable include, but are not limited to, fluorescent labels (e.g., fluorescein isothiocyanate, texas red, rhodamine, a green fluorescent protein, a red fluorescent protein, a yellow fluorescent protein, and the like), radiolabels (e.g., ³H, ¹²⁵I, ³⁵S, ¹⁴C, or ³²P), and enzymes (e.g., horse radish peroxidase, alkaline phosphatase, luciferase, and other enzymes that act on a substrate to produce a product that can be detected by fluorometric, colorimetric, or spectrophotometric means).

As indicated above, a subject kit can include standard prebeta-1 HDL of greater than 90% purity, greater than 95% purity, greater than 98% purity, or greater than 99% purity; or includes a surrogate for preβ-1 HDL. The standard preβ-1 HDL can be prepared from fresh normal human plasma by selected affinity immunosorption, or any other suitable method. Thus a subject kit includes purified preβ-1 HDL suitable for generating a standard curve, e.g., for quantitating the preβ-1 HDL detected in a test biological sample from a test individual. Examples of amounts of preβ-1 HDL suitable for generating a standard curve include, e.g., 0.5 µg, 1.0 µg, 1.5 µg, 2.0 µg, and 5.0 µg. A subject kit can include purified preβ1-HDL in a concentration such that dilutions can be made and applied to a gel to provide for suitable quantities to generate a standard curve.

A kit will include one or more components for carrying out gel electrophoresis. For example, the kit can include components for making a gel, where such components include, e.g., agarose or polyacrylamide; a buffer; etc. A kit can further include components such as reagents for detecting apoA-I-antibody complexes present in a gel. For example, a kit can include Coomassie blue or other suitable dye.

Other optional components of the kit include: a buffer; a detectable label; components for developing a detectable label (e.g., where the antibody reagent includes a detectable label); etc. The various components of the kit may be present in separate containers or certain compatible components may be pre-combined into a single container, as desired.

In addition to above-mentioned components, a subject kit can include instructions for using the components of the kit to practice a subject method. The instructions for practicing a subject method are generally recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or subpackaging) etc. In other embodiments, the instructions are present as an electronic storage data file present on a suitable computer readable storage medium, e.g. compact disc-read only memory (CD-ROM), digital versatile disk (DVD), diskette, etc. In yet other embodiments, the actual instructions are not present in the kit, but means for obtaining the instructions from a remote source, e.g. via the internet, are provided. An example of this embodiment is a kit that includes a web address where the instructions can be viewed and/or from which the instructions can be downloaded. As with the instructions, this means for obtaining the instructions is recorded on a suitable substrate.

### EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Celsius, and pressure is at or near atmospheric. Standard abbreviations may be used, e.g., bp, base pair(s); kb, kilobase(s); pl, picoliter(s); s or sec, second(s); min, minute(s); h or hr, hour(s); aa, amino acid(s); kb, kilobase(s); bp, base pair(s); nt, nucleotide(s); i.m., intramuscular(ly); i.p., intraperitoneal(ly); s.c., subcutaneous(ly); and the like.

### Example 1: Association of increased preβ-1 HDL levels with risk of cardiovascular disease

### METHODS

### Subjects and study design

Subjects referred because of demonstrable CAD or for risk factor assessment and management to the University of California at San Francisco (UCSF) Lipid Clinic, the UCSF Division of Cardiology, and to collaborating cardiology practices were recruited for the study. All patients were clinically stable at the time of recruitment. The study was approved by the Committee on Human Research of UCSF and written consent was obtained from all subjects. Due to the potentially confounding effect of lipid-lowering medications, subjects with missing information regarding medication were excluded (n=1,048), leaving 1,255 available for analyses. Subjects were considered to have CAD if any of the following criteria were fulfilled: coronary atherosclerosis demonstrable by angiography, myocardial infarction, coronary revascularization, angioplasty, or stent placement. The four principal coronary vessels and their branches were imaged in all angiographic studies after administration of nitroglycerin. Coronary disease on angiography (n=296 individuals) was graded by a "sum score", defined as the sum of the measured percent luminal intrusion of all coronary lesions. A consensus sum score of 60 was chosen as the threshold discriminant for the diagnosis of coronary artery disease in this study. The mean sum score was 240.6. The diagnosis of MI was based on at least one of the following: troponin levels, electrocardiographic evidence, or echocardiographic studies.

### Measurement of lipids and lipoproteins

### Sample collection and measurement of major lipoprotein classes:

Measurement of plasma lipids and major lipoprotein classes was made on blood samples drawn after a minimum ten hour fast. All samples were drawn into tubes containing buffered K2-EDTA (Becton Dickinson Vacutainer), and were chilled immediately in ice to stop esterification of free cholesterol by LCAT. This is essential to prevent conversion of preβ-1 HDL to larger HDL species, a process that proceeds at room temperature with a half-life of approximately 50 minutes. Ishida et al. (1990) J. Lipid Res. 31:227; Miida et al. (2003) J. Am. Soc. Nephrol. 14:732. Major lipoprotein lipids were measured on lipoprotein fractions isolated by ultracentrifugation (Pullinger et al. (1995) J. Clin. Invest. 95:1225) in 65% of subjects (including all subjects with triglycerides in excess of 300 mg/dL), or in plasma after precipitation of the apoB containing lipoproteins with dextran sulfate. Warnick et al. (1982) Clin. Chem. 28:1379. In the latter case, low density lipoprotein (LDL) cholesterol content was calculated by the Friedewald formula. Friedewald et al. (1972) Clin. Chem. 18:499. Subjects with plasma triglycerides exceeding 500 mg/dL were excluded to avoid confounding alterations in the molecular speciation of HDL. Lipid measurements were standardized against reference material supplied by the Standardization Program of the National Center for Disease Control.

### Measurement of prebeta-1 HDL:

An independent aliquot of plasma was placed immediately in ice and then frozen at -80° C for measurement of the apoA-I content of preβ-1 HDL by the technique of ultrafiltration-isotope dilution, previously developed in our laboratory. O'Connor et al. (1997) Anal. Biochem. 251:234. Briefly, is was previously shown that prebeta-1 HDL can be categorically separated from larger diameter HDL particles by low velocity centrifugation in ultrafilters and that it can be measured in plasma by application of the isotope dilution principle. O'Connor et al. (1997) supra. The ultrafiltered HDL particles show discrete prebeta-1 mobility by Western blotting in agarose gel electrophoresis identical to that when 2-dimensional electrophoresis is performed on fresh plasma. The ultrafiltered prebeta-1 HDL particle diameters as measured in the second dimension of 2-D gel electrophoresis are also identical with those of prebeta-1 HDL studied directly from plasma. Additionally, the particle diameters are consistent with a molecular mass circa 60 kDa as confirmed by FPLC analysis. O'Connor et al. (1997) *supra.* Thus it is believedd that the ultrafiltration technique measures the identical molecular subspecies of HDL that can be measured by Western blotting of 2-dimensional electrophoretograms. It has been found that these characteristics of the prebeta-1 HDL particle are consistent in patients with major dyslipidemic phenotypes with the exception of extreme hypertriglyceridemia (triglyceride levels in excess of 500 mg/dL.) where aberrant prebeta HDL particles may be observed with those of prebeta-1 HDL. Such subjects were excluded from this cohort. For this analysis, standard prebeta-1 HDL of greater than 99% purity, prepared from fresh normal human plasma by selected affinity immunosorption (McVicar et al. (1984) Proc. Natl. Acad. Sci. USA 81:1356) and preparative electrophoresis is labeled with a tritium adduct. A precisely measured amount is added to the patient's plasma sample. The content of apoA-I in the ultrafiltrate is measured by ELISA (Kunitake et al. (1996) Methods Enzymol. 263:260) for calculation of its specific activity. The counts applied in the sample divided by the specific activity yields the content of prebeta-1 HDL in the original sample. The assays are run in duplicate with intra- and inter-assay coefficients of variation of 6% and 7% respectively. Samples are stable for extended periods of time at -80 degrees C. The incremental addition of purified prebeta-1 HDL to plasma results in linear recovery by this method. The relationships of prebeta-1 HDL levels to clinical and biochemical variables have been reported in normolipidemic individuals. O'Connor et al. (1998) J. Lipid Res. 39:670.

### Statistical Methods

Descriptive statistics of demographic and clinical characteristics are presented as mean ± standard deviation or median (median absolute deviation) for continuous variables and number of patients (percentages) for categorical variables. Percent preβ-1 HDL level, defined as the percent of total plasma apoA-I in preβ-1 HDL, was used in all analyses. Percent of preβ-1 HDL was categorized into tertiles and the distribution of traditional cardiovascular risk factors were compared across tertile groups. Associations between preβ-1 HDL tertiles and cardiovascular risk factors were examined using analysis of variance for continuous variables and chi-square test of independence for categorical variables.

To evaluate the association of preβ-1 HDL with CAD and MI, unadjusted and adjusted odds ratios (OR) and their 95% confidence intervals (CI) were estimated for preβ-1 HDL in univariable and multivariable logistic regression models respectively. The multivariable logistic regression models included conventional non-lipid cardiovascular risk factors (age, gender, race, body mass index [BMI], smoking status, diabetes, and hypertension), lipid risk factors (HDL, LDL, and triglycerides [TG]), and reported lipid-altering medication usage. Continuous variables with positively skewed distributions (preβ-1 HDL, BMI, HDL, and LDL) were log-transformed and standardized. Thus, their ORs represent the increased odds of disease prevalence given a 1 standard deviation increment. Similarly, age was divided into 5-year intervals such that its OR represents the increased odds of disease risk given a 5-year increase in age. Due to the highly skewed nature of TG, levels were dichotomized at 200 mg/dl in the regression models. Interactions between preβ-1 HDL and gender were also examined. Multivariable models also were estimated in a subgroup of patients who did not report using lipid-lowering medications (n=1,063).

To evaluate the contributions of preβ-1 HDL to risk models, likelihood-ratio tests (LRT) were performed comparing risk models with and without the inclusion of preβ-1 HDL as a predictor. Additionally, the improvement in area under the receiver-operating-characteristic (ROC) curves (AUC) following the addition of preβ-1 HDL in multivariable risk models was evaluated. Because improvements in AUC can be rather insensitive to biomarkers with moderate effect sizes and even established cardiovascular risk factors such as lipids, hypertension, and smoking (Pencina et al. (2008) Stat. Med. 27:157; and Cook (2007) Circulation 115:928), the improvement in classification of cases and controls due to addition of preβ-1 HDL was also evaluated in multivariable risk models with the two measures: (1) the net reclassification index (NRI) using predefined risk categories of <6%, 6-20% and >20% and (2) the integrated discrimination improvement (IDI) index. The NRI classifies patients into risk categories defined *a priori* in a model with and without a biomarker and formally tests whether there was a net gain in reclassification due to the biomarker. A gain in reclassification occurs when cases are reclassified into higher risk categories and controls are reclassified into lower risk categories. Conversely, a loss in reclassification occurs when cases are reclassified into lower risk categories and controls are reclassified into higher risk categories. These gains and losses are subsequently aggregated to assess statistically whether there was a net gain in reclassification due to a biomarker. Pencina et al. (2008) *supra.* Because the NRI is dependent upon predefined risk categories, we also evaluated the improvement in classification due to preβ-1 HDL with the IDI which does not require predefined risk categories. Pencina et al. (2008) *supra.*

Results with p<0.05 were considered to be statistically significant. All analyses were performed in R version 2.9.0.

### RESULTS

### Patient characteristics

Descriptive characteristics of the 1,255 subjects according to preβ-1 HDL tertiles are presented in Table 1. The onset of clinical coronary disease was relatively early among the affected subjects (mean age at time of study was 60.2 years). Less than half of the subjects were male (44.5%) and the average BMI was 26.7. The majority were of European ancestry (73.4%), followed by Asian ancestry (15.6%), and the remainder were distributed among African-American and Hispanic ancestry. Type 2 diabetes was present in 8.4% and 6.6% were present smokers.

**Table 1: Demographic and clinical characteristics according to tertiles of preβ-1 HDL**

| | Preβ-1 HDL Tertiles | | | |
|---|---|---|---|---|
| | <4.171 (n=418) | 4.171-8.838 (n=418) | >8.838 (n=419) | *P* |
| Age | 52.24±17.08 | 51.2±16.99 | 48.94±16.61 | 0.005 |
| Men | 155 (37.1 %) | 201 (48.1 %) | 202 (48.2%) | <0.001 |
| Body mass index (kg/m2) | 25.22±4.95 | 27.17±5.81 | 27.34±5.12 | <0.001 |
| Race | | | | 0.015 |
| Caucasian | 287 (70.0%) | 299 (73.6%) | 315 (76.6%) | |
| Asian | 84 (20.5%) | 57 (14.0%) | 51 (12.4%) | |
| Other | 39 (9.5%) | 50 (12.3%) | 45 (10.9%) | |
| Hypertension | 123 (29.5%) | 138 (33.0%) | 161 (38.4%) | 0.023 |
| Smoker (current) | 16 (3.8%) | 25 (6.0%) | 42 (10.0%) | 0.001 |
| Lipid lowering medication | 68 (16.3%) | 61 (14.6%) | 64 (15.3%) | 0.796 |
| Triglycerides (mg/dl) | 109 (56.3) | 151 (77.1) | 199 (147.8) | <0.001 |
| LDL cholesterol (mg/dl) | 146.52±53.27 | 148.74±56.17 | 148.51±65.82 | 0.50 |
| HDL cholesterol (mg/dl) | 58.39±19.79 | 48.44±16.78 | 45.49±14.69 | <0.001 |
| apoA-I (mg/dl) | 1.22±0.35 | 1.15±0.38 | 1.14±0.31 | <0.001 |
| Type 2 Diabetes | 32 (7.7%) | 28 (6.7%) | 45 (10.7%) | 0.088 |
| Coronary artery disease | 126 (30.1 %) | 158 (37.8%) | 170 (40.6%) | 0.005 |
| Myocardial infarction | 56 (13.4%) | 68 (16.3%) | 89 (21.2%) | 0.0097 |

The distributions of demographic and clinical characteristics are shown across tertiles of preβ-1 HDL, defined as percent of total plasma apoA-I present in prebeta-1 HDL. Data are presented as number of subjects (percentage), mean ± SD or median (median absolute deviation). The number of subjects for each demographic and clinical characteristic, including race, varies due to missing data.

The mean±SD preβ-1 HDL was 7.87±6.45 percent of plasma apoA-I in the entire study cohort. Preβ-1 HDL levels were associated with all demographic and clinical characteristics with the exception of LDL cholesterol, lipid-lowering medication, and diabetes. Relatively few subjects were receiving lipid-lowering medications at the time of referral. Because virtually all the blood samples were drawn at initial visits, and because many subjects were asymptomatic, lipid lowering drug therapy had not yet been initiated by the referring physicians. Of the 16% taking lipid-lowering drugs at entry, 64% of these had been prescribed a statin, 8% were taking niacin, and the remainder were taking a combination of a statin with a resin or fibrate. One subject was taking ezetimibe. It is unlikely that lipid lowering medications will have a significant impact on the finding of this study based on the observation that a comparison of prebeta-1 HDL values in patients taking those medications with those who were not did not reveal a significant difference in the prebeta-1 HDL levels. (Table 2.)

**Table 2: Predictors of CAD and MI in the entire population and in a subset of individuals not using lipid-lowering medication**

| | CORONARY ARTERY DISEASE | | | | MYOCARDIAL INFARCTION | | | |
|---|---|---|---|---|---|---|---|---|
| | All individuals (n=1164) | | Individuals not using lipid-lowering medication (n=989) | | All individuals (n=1163) | | Individuals not using lipid-lowering medication (n=989) | |
| | OR (95% CI) | p Value | OR (95% CI) | p Value | OR (95% CI) | p Value | OR (95% CI) | p Value |
| Preβ-1 HDL tertiles | | | | | | | | |
| <4.171 | 1.00 (reference) | --- | 1.00 (reference) | --- | 1.00 (reference) | --- | 1.00 (reference) | --- |
| 4.171-8.838 | 1.34 (0.92-1.96) | 0.128 | 1.38 (0.89-2.14) | 0.147 | 1.17 (0.75-1.83) | 0.491 | 1.22 (0.71-2.08) | 0.475 |
| >8.838 | 1.65 (1.11-2.45) | 0.014 | 1.65 (1.04-2.61) | 0.034 | 1.92 (1.22-3.02) | 0.005 | 1.69 (0.98-2.91) | 0.058 |
| | LRT=12.8 | 0.002 | LRT=9.44 | 0.009 | LRT=19.4 | <0.001 | LRT=7.88 | 0.019 |
| Age (5 years) ‡ | 1.37 (1.29-1.45) | <0.001 | 1.39 (1.29-1.48) | <0.001 | 1.2 (1.13-1.29) | <0.001 | 1.18 (1.1-1.28) | <0.001 |
| Men | 0.4 (0.28-0.57) | <0.001 | 0.29 (0.2-0.44) | <0.001 | 0.62 (0.41-0.92) | 0.017 | 0.47 (0.29-0.76) | 0.002 |
| BMI † | 1.12 (0.95-1.32) | 0.177 | 1.12 (0.93-1.35) | 0.244 | 0.82 (0.67-1.01) | 0.051 | 0.83 (0.66-1.03) | 0.092 |
| Race | | | | | | | | |
| Caucasian | 1.00 (reference) | --- | 1.00 (reference) | --- | 1.00 (reference) | --- | 1.00 (reference) | --- |
| Asian | 0.69 (0.44-1.09) | 0.109 | 0.68 (0.39-1.18) | 0.166 | 0.52 (0.29-0.93) | 0.027 | 0.57 (0.28-1.16) | 0.122 |
| Other | 0.76 (0.46-1.25) | 0.278 | 0.66 (0.36-1.21) | 0.179 | 0.86 (0.48-1.51) | 0.591 | 0.69 (0.33-1.44) | 0.323 |
| Hypertension | 3.17 (2.32-4.34) | <0.001 | 4.39 (3.05-6.32) | <0.001 | 2.55 (1.77-3.69) | <0.001 | 3.47 (2.25-5.36) | <0.001 |
| Smoker (current) | 1.7 (0.97-2.97) | 0.062 | 1.77 (0.93-3.37) | 0.082 | 0.94 (0.49-1.8) | 0.856 | 0.94 (0.44-1.98) | 0.865 |
| Type 2 Diabetes | 0.78 (0.46-1.3) | 0.339 | 0.64 (0.33-1.25) | 0.193 | 1.23 (0.72-2.12) | 0.453 | 1.39 (0.68-2.84) | 0.363 |
| HDL † | 0.57 (0.47-0.7) | <0.001 | 0.53 (0.42-0.66) | <0.001 | 0.65 (0.53-0.81) | <0.001 | 0.63 (0.49-0.81) | <0.001 |
| LDL † | 0.96 (0.82-1.12) | 0.592 | 0.98 (0.83-1.17) | 0.858 | 0.94 (0.79-1.13) | 0.535 | 1.03 (0.84-1.28) | 0.755 |
| High TG (>200 mg/dl) | 0.69 (0.48-0.99) | 0.044 | 0.69 (0.45-1.05) | 0.086 | 0.74 (0.49-1.12) | 0.159 | 0.84 (0.51-1.36) | 0.467 |
| Lipid Rx | 3.31 (2.15-5.09) | <0.001 | --- | --- | 2.23 (1.42-3.5) | <0.001 | --- | --- |

| | | | | | | | | |
|---|---|---|---|---|---|---|---|---|
| †Per standard deviation in ‡Per 5-year increment. | | | | | | | | |

Subjects with elevated preβ-1 HDL were slightly younger but had more adverse clinical characteristics in general. Specifically, they had higher BMIs, higher TG levels, lower HDL cholesterol levels, lower apoA-1 levels, and were more likely to be males, current smokers and hypertensive. The negative correlation between preβ-1 HDL and HDL cholesterol was more pronounced in patients without CAD (*r*=-0.34, p<0.001) than patients with CAD (*r*=-0.16, p<0.001). Additionally, preβ-1 HDL differed among races with Asians having significantly lower preβ-1 HDL than other race designations reported in this study. Although menopausal status was not obtained from patients, preβ-1 HDL levels were examined in women older than 55 years (who are most likely post-menopausal, n=212) and women younger than 35 years (who are most likely pre-menopausal, n=132). Preβ-1 HDL levels were similar between these two age groups (pre-menopausal mean±SD: 6.84±6.07 vs. post-menopausal mean±SD: 6.92±5.91, P=0.78).

### Association of preβ-1 HDL with CAD and MI

Unadjusted and adjusted associations of preβ-1 HDL with CAD and MI are presented in Table 3. The prevalence of CAD and MI in the study population was 36.2% (454/1255) and 17.0% (213/1254) respectively. Preβ-1 HDL levels were significantly and positively associated with increased risk of both CAD and MI, even after adjustment for conventional risk factors. Likelihood-ratio tests show that preβ-1 HDL contributed significantly to both unadjusted and adjusted models. Estimates for all covariates entered in multivariable risk models were obtained. Results were similar when the analysis was limited to the subgroup of patients who were not using lipid-lowering medication (Table 2).

**Table 3: Associations of preβ-1 HDL tertiles with coronary artery disease and myocardial infarction**

| | Coronary artery disease | | Myocardial infarction | |
|---|---|---|---|---|
| | OR (95% CI) | *P* | OR (95% CI) | *P* |
| *UNADJUSTED* | | | | |
| Preβ-1 HDL tertiles | | | | |
| <4.171 | 1.00 (reference) | --- | 1.00 (reference) | --- |
| 4.171-8.838 | 1.41 (1.06-1.88) | 0.020 | 1.25 (0.85-1.84) | 0.249 |
| >8.838 | 1.58 (1.19-2.11) | 0.002 | 1.74 (1.21-2.51) | 0.003 |
| Likelihood-ratio test | 21.4 | <0.001 | 18.4 | <0.001 |
| | | | | |

| *ADJUSTED* | | | | |
|---|---|---|---|---|
| Preβ-1 HDL tertiles | | | | |
| <4.171 | 1.00 (reference) | --- | 1.00 (reference) | --- |
| 4.171-8.838 | 1.35 (0.92-1.96) | 0.124 | 1.17 (0.75-1.83) | 0.483 |
| >8.838 | 1.64 (1.1-2.44) | 0.015 | 1.91 (1.22-3.00) | 0.005 |
| Likelihood-ratio test | 12.8 | 0.002 | 19.4 | <0.001 |

Unadjusted and adjusted odds ratios (OR) and their 95% confidence intervals (CI) are shown for tertiles of preβ-1 HDL defined as percent of total apoA-I present in plasma. Adjusted odds ratios adjust for age, gender, BMI, race, hypertension, current smoking status, Type 2 diabetes, HDL cholesterol, LDL cholesterol, TG and usage of lipid-lowering medication in multivariable logistic regression models. Likelihood-ratio tests were performed comparing nested models with and without the inclusion of preβ-1 HDL tertiles in logistic regression models.

### Improvement in classification due to preβ-1 HDL

Table 4 shows three statistical measures that were used to evaluate the utility of adding preβ-1 HDL as an additional predictor in conventional risk factor models. Inclusion of preβ-1 HDL in multivariable models led to trivial increments in the AUC for both CAD and MI. However, changes in AUC are known to be insensitive to biomarkers of moderate effect. The incorporation of preβ-1 HDL into the risk model for CAD resulted in a significant improvement in classification as indicated by the NRI of 14.9% (95% CI=2.9%-26.9%, p=0.015); however the IDI estimate was non-significant, suggesting that the reclassification improvement was not robust to differing definitions of risk categories. In contrast, incorporation of preβ-1 HDL into the risk model for MI led to a significant net reclassification gain of 21.3% (95% CI=5.8%-36.7%, p=0.007) and a significant IDI of 1% (p=0.012).

**Table 4: Effect of adding preβ-1 HDL tertiles to risk prediction models**

| | AUC | | NRI | | IDI | |
|---|---|---|---|---|---|---|
| | Change | *P* | Estimate | *P* | Estimate | *P* |
| Coronary artery disease | 0.001 | 0.476 | 0.149 | 0.015 | 0.003 | 0.135 |
| Myocardial infarction | 0.006 | 0.198 | 0.213 | 0.007 | 0.010 | 0.012 |

| | | | | | | |
|---|---|---|---|---|---|---|
| AUC=area under the receiver operating curve; NRI=net reclassification improvement; IDI=integrated discrimination improvement | | | | | | |

The increment due to the inclusion of preβ-1 HDL tertiles in risk models compared with conventional risk factors alone is shown for various calibration statistics. The AUC in risk models that exclude preβ-1 HDL is 0.845 for coronary artery disease and 0.782 for myocardial infarction.

### Example 2: Immunofixation assay for preβ-1 HDL

Human blood acquired by venipunture into potassium-EDTA (Becton-Dickinson Vacutainers) is immediately cooled to 0° C in an ice water bath. Plasma is prepared by centrifugation (3,000 x g, 30', 3°C) for preβ-1 HDL assay or aliquoted (1 ml) and quickly frozen in dry ice for storage at -80°C. Stored frozen plasma samples are quickly thawed in a circulating water bath at 37°C for 2 minutes and placed on ice.

Urea-disrupted HDL is used as a surrogate electrophoretic marker and apoAI mass calibrator for preβ-1 HDL in plasma samples. Urea-disrupted HDL is prepared from HDL prepared by sequential potassium bromide density gradient ultracentrifugation (1.063 < d < 1.21 g/ml) of human plasma acquired from healthy subjects. The isolated HDL is extensively dialyzed against buffer (0.15 M NaCl, 0.001 M Na-EDTA, 0.02% sodium azide). HDL is characterized for total protein content (BioRad DC Kit, bovine serum albumin standard) and its fractional apoAI content determined as the percent area of peaks scanned and integrated (BioRad Chemdoc, Image Lab software) corresponding to the Coomassie blue R-250 stained apoAI band (Mᵣ 25 kDa) on sodium docecyl sulfate-polyacrylamide gel electrophoresis (SDS-PAGE). Stock HDL (appr. 5 mg/ml) solutions are disrupted by the addition of urea buffer (6M urea, 0.02 M tris, 0.001 M EDTA, 0.02% sodium azide) to effect a final apoAI concentration of 0.133 micrograms/ml. The disrupted HDL solution is serial two-fold diluted in urea buffer to effect solutions of urea-disrupted HDL (apoHDL) containing the apoAI equivalents of 133 mg/dL, 67 mg/dL, 33 mg/dL, 17 mg/dL, and 8 mg/dL.

Electrophoretic resolution of plasma samples and the application of mono-specific antisera in agarose gels are conducted in a semi-automated electrophoretic apparatus (SPIFE, Model 3000, Helena Laboratories, Beaumont, TX).

After electrophoresis, the gel is contacted with an antibody that specifically binds apoA-I (e.g., apoA-I present in preβ1-HDL). The antibody enters the gel and forms complexes with apoA-I-containing molecules present in the biological sample, thereby generating apoA-I-antibody complexes. The apoA-I-antibody complexes are large and are therefore "fixed" within the gel, e.g., the apoA-I-antibody complexes are not readily removed from the gel matrix. The fixation process can occur when antibody comes in contact with apoA-I-containing molecules at temperatures ranging from 3°C to 37° C over a period of time ranging from several minutes (e.g., from about 5 minutes to about 120 minutes) to hours (e.g., from about 2 hours to about 4 hours, from about 4 hours to about 6 hours, from about 6 hours to about 8 hours), or longer than 8 hours. The antibody can be applied in a liquid medium in the form of purified antibody suspended in an appropriate buffer solution or as antisera (e.g. monospecific goat antiserum) at neat or diluted in an appropriate buffer solution. The antibody-containing medium may be used as such or used with additives, including but not limited to, a non-ionic detergent (e.g., Triton X-100, Tween-20, and NP-40), a protease inhibitor, and the like.

While the present invention has been described with reference to the specific embodiments thereof, it should be understood by those skilled in the art that various changes may be made and equivalents may be substituted.

The invention is defined by the accompanying claims.

## Claims

1. A method of identifying an individual at risk of having a myocardial infarction, the method comprising:
determining the level of pre-beta-1 high density lipoprotein (preβ-1 HDL) in a biological sample from the individual,
wherein a level of preβ-1 HDL that is higher than a normal control level indicates that the individual is at increased risk of having a myocardial infarction.

2. The method of claim 1, wherein the biological sample is blood or a blood product, wherein the blood product is optionally plasma or serum.

3. The method of claim 1 or 2, wherein the individual has or is suspected of having a cardiovascular disease.

4. The method of claim 3, wherein the cardiovascular disease is atherosclerosis, coronary artery disease, myocardial infarction, angina, stroke, or heart failure.

5. The method of claim 1 or 2, wherein the individual is free of symptoms of a cardiovascular disease.

6. The method of any one of any one of claims 1 to 5, wherein said determining is carried out using an immunological assay, which optionally is an immunofixation assay.

7. The method of claim 6 wherein the immunofixation assay comprises:
a) electrophoretically separating components of the biological sample in a gel;
b) contacting the gel with an antibody that specifically binds apolipoprotein A-I (apoA-I), wherein said antibody enters the gel and forms complexes with apoA-I-containing molecules present in the biological sample, thereby generating apoA-I-antibody complexes; and
c) detecting the apoA-I-antibody complexes in the gel; and
d) comparing the level of detected apoA-I-antibody complexes with the level of purified preβ-1 HDL standards present in the gel to measure a level of preβ-1 HDL present in the biological sample.

8. The method of claim 7, further comprising, between step (b) and step (c), a step of removing uncomplexed components from the gel.

9. The method of claim 7 or 8, wherein the antibody is specific for preβ-1 HDL.

10. The method of any one of claims 7 to 9, wherein said detecting comprises staining the apoA-I-antibody complexes.

11. The method of any one of claims 7 to 10, wherein the antibody comprises a detectable label.

## Patentansprüche

1. Verfahren zur Identifizierung eines Individuums, bei dem das Risiko für einen Myokardinfarkt besteht, wobei das Verfahren Folgendes umfasst:
das Ermitteln des Spiegels von hochdichtem Prä-β-1-Lipoprotein (Prä-β-1-HDL) in einer biologischen Probe von dem Individuum,
wobei ein Spiegel von Prä-β-1-HDL, der höher ist als ein normaler Kontrollspiegel, anzeigt, dass bei dem Individuum ein erhöhtes Risiko für einen Myokardinfarkt besteht.

2. Verfahren nach Anspruch 1, wobei die biologische Probe Blut oder ein Blutprodukt ist, wobei das Blutprodukt gegebenenfalls Plasma oder Serum ist.

3. Verfahren nach Anspruch 1 oder 2, wobei das Individuum an einer Herz-Kreislauf-Erkrankung leidet oder ein dahingehender Verdacht besteht.

4. Verfahren nach Anspruch 3, wobei die Herz-Kreislauf-Erkrankung Atherosklerose, Koronararterienerkrankung, Myokardinfarkt, Angina, Schlaganfall oder Herzversagen ist.

5. Verfahren nach Anspruch 1 oder 2, wobei das Individuum keine Symptome einer Herz-Kreislauf-Erkrankung aufweist.

6. Verfahren nach einem der Ansprüche 1 bis 5, wobei das Bestimmen unter Anwendung eines immunologischen Tests erfolgt, der gegebenenfalls ein Immunfixationstest ist.

7. Verfahren nach Anspruch 6, worin der Immunfixationstest Folgendes umfasst:
a) elektrophoretisches Trennen von Komponenten der biologischen Probe in einem Gel;
b) Kontaktieren des Gels mit einem Antikörper, der Apolipoprotein A-I (ApoA-I) spezifisch bindet, wobei der Antikörper in das Gel eintritt und mit in der biologischen Probe vorhandenen ApoA-I-Antikörper-hältigen Molekülen Komplexe bildet, wodurch ApoA-I-Antikörper-Komplexe gebildet werden; und
c) Detektieren der ApoA-I-Antikörper-Komplexe in dem Gel; und
d) das Vergleichen der Menge an detektierten ApoA-I-Antikörper-Komplexen mit der Menge an in dem Gel vorhanden, gereinigten Prä-β-1-HDL-Standards, um den Prä-β-1-HDL-Spiegel in der biologischen Probe zu messen.

8. Verfahren nach Anspruch 7, das weiters zwischen Schritt (b) und Schritt (c) einen Schritt des Entfernens von nichtkomplexierten Komponenten aus dem Gel umfasst.

9. Verfahren nach Anspruch 7 oder 8, wobei der Antikörper für Prä-β-1-HDL spezifisch ist.

10. Verfahren nach einem der Ansprüche 7 bis 9, wobei das Detektieren das Färben der apoA-I-Antikörper-Komplexe umfasst.

11. Verfahren nach einem der Ansprüche 7 bis 10, wobei der Antikörper eine detektierbare Markierung umfasst.

## Revendications

1. Procédé d'identification d'un individu présentant un risque d'avoir un infarctus du myocarde, le procédé comprenant les étapes consistant à :
déterminer le niveau de lipoprotéine pré-bêta-1 (preβ-1 HDL) haute densité dans un échantillon biologique provenant de l'individu,
dans lequel un niveau de preβ-1 HDL qui est supérieur à un niveau témoin normal indique que l'individu présente un risque accru d'avoir un infarctus du myocarde.

2. Procédé selon la revendication 1, dans lequel l'échantillon biologique est du sang ou un produit sanguin, dans lequel le produit sanguin est du plasma ou du sérum.

3. Procédé selon la revendication 1 ou 2, dans lequel l'individu a ou est suspecté d'avoir une maladie cardiovasculaire.

4. Procédé selon la revendication 3, dans lequel la maladie cardiovasculaire est l'athérosclérose, une maladie coronarienne, l'infarctus du myocarde, une angine, un accident vasculaire cérébral ou une insuffisance cardiaque.

5. Procédé selon la revendication 1 ou 2, dans lequel l'individu est exempt de symptômes d'une maladie cardiovasculaire.

6. Procédé selon l'une quelconque des revendications 1 à 5, dans lequel ladite détermination est effectuée en utilisant un dosage immunologique, qui est facultativement un essai d'immunofixation.

7. Procédé selon la revendication 6, dans lequel l'essai d'immunofixation comprend :
a) la séparation par électrophorèse de composants de l'échantillon biologique dans un gel ;
b) la mise en contact du gel avec un anticorps qui se lie spécifiquement à l'apolipoprotéine A-I (apoA-I), ledit anticorps entrant dans le gel et formant des complexes avec des molécules contenant de l'apoA-I présentes dans l'échantillon biologique, en générant ainsi des complexes apoA-I-anticorps ; et
c) la détection des complexes apoA-I-anticorps dans le gel ; et
d) la comparaison du niveau des complexes apoA-I-anticorps détectés avec le niveau de normes de preβ-1 HDL purifiée présent dans le gel pour mesurer un niveau de preβ-1 HDL présent dans l'échantillon biologique.

8. Procédé selon la revendication 7, comprenant en outre, entre l'étape (b) et l'étape (c), une étape d'élimination des composants non complexés à partir du gel.

9. Procédé selon la revendication 7 ou 8, dans lequel l'anticorps est spécifique pour preβ-1 HDL.

10. Procédé selon l'une quelconque des revendications 7 à 9, dans lequel ladite détection comprend la coloration des complexes apoA-I-anticorps.

11. Procédé selon l'une quelconque des revendications 7 à 10, dans lequel l'anticorps comprend un marqueur détectable.
